# EUROPEAN PATENT APPLICATION

(11) **EP 2 799 062 A1**
(43) Date of publication of application: **05.11.2014**
(21) Application number: 13002357.5
(22) Date of filing: 03.05.2013
(51) Int. Cl.: A61K 9/00, A61K 9/28, A61K 9/50, A61K 35/00

(54) **Pharmaceutical product for inducing magnified release of endogenous gut hormones, pyy and glp-1, to a therapeutic effect on obesity and dm2**

(71) Applicant: Brito, Gilberto Borges de, 15061-900 Sao Paulo (BR)
(72) Inventor: Brito, Gilberto Borges de, 15061-900 Sao Paulo (BR)
(74) Representative: Pereira Garcia, João Luís

(57) **Abstract**

Pharmaceutical product for inducing magnified endogenous release of gut hormones, PYY and GLP-1, to reach a therapeutic effect for obesity and type 2 Diabetes Mellitus, prepared from an associative formula of macro and micronutrients in tablet forms or pills or mini-pills or micro-granules or micronized particles to be coated or microencapsulated with polymers or coating products or substances or microencapsulating products or substances, which, after ingestion, travels unchanged through the esophagus into the stomach, duodenum, and jejunum. The product was developed for travelling through the gastrointestinal tract, and, only after reaching the ileum, will suffer disintegration or breaking up of its coating, because of the specific pH encountered there, in the range of 6,0 to 7,0, then releasing its contents to cause an amplified stimulus on L cells, therein encountered in their highest concentration, to secrete the endogenous gut hormones PYY and GLP-1, which, through the bloodstream reach the arched nucleus in the hypothalamus (brain), causing intense inhibition of appetite, inducing the patient to reduce food intake and also stimulating the pancreatic Beta Cells (GLP-1) to secrete insulin while inhibiting pancreatic Alfa Cells in releasing glucagon, effects that results in better glycemic control in type 2 diabetic patients. Due to the product in question and the stimuli produced by it, beneficial effects of anti-obesity and control of Diabetes Mellitus 2 are obtained, presumably in similar ways as do bariatric-metabolic surgery, but without their inherent risks and potential early and late complications and, at the same time, facilitating the access of the massive numbers of individuals worldwide, suffering from obesity and/or type 2 diabetes mellitus to these benefits, because of the method simplicity.

## Description

Refers to this descriptive report, a patent application of the invention of a product to induce endogenous releasing of gut hormones, mainly and specially the PYY and GLP-1, by means of delivering a mixture of undigested or partially digested macronutrients with or without micronutrients and bile salts, in any effective associative composition, directly in the ileum and/or colon. The nutrient mixture is compressed in tablet forms or in micro-granules or in micronized particles followed by a special coating for a pH dependent release of its contents. in this system, whatever the coating product or polymer or substance is used, it has to disintegrate or dissolve or breakup within a ph range from 6,0 to 7,0, for accomplishing the release of the associative composition of the substances above mentioned, in the ileum and/or colon, there stimulating the secretion of gut hormones in an amplified or supra-physiological manner, mainly PYY and GLP-1, which in high blood levels, will produce intense appetite inhibition and increase energy expenditure for an anti-obesity and its associated co-morbidities effect, and also, BY stimulating the GLP-1-mediated insulin secretion and glucagon inhibition, for an anti-type 2 diabetes mellitus effect.

### STATE-OF-THE-SCIENCE

As it is known, obesity is associated with diseases such as diabetes, hypertension, dyslipidemia, obstructive sleep apnea, steatohepatitis, increased risk by 3 to 5 times for various types of cancer and numerous other illnesses that cause severe consequences, such as a mortality rate of 7 to 10 times larger than in normal weight populations, in addiction to drastic loss in quality of life and low self-esteem.

Bariatric-metabolic surgeries comprise the most effective means to solve the obesity and the serious health problems (cited above) associated with it, where by means of intestinal diversions, such as in Fobi-Capella surgery, also known as roux-en-y gastric bypass or in surgeries named as bileo-pancreatic diversion, such as Scopinaro's surgery and duodenal switch surgery, expose the distal portions of the small intestine, called ileum, and/or the proximal colon, to food components, undigested or partially digested, which stimulates the release of hormones, such as PYY and OLP-1 (as shown in the attached plot, included as Figure I) responsible, in great extent, to the success of these surgeries, thereby helping to control severe obesity in the long run, in approximately 80 % of the morbidly obese patients and type 2 diabetes mellitus in 90-95% of these patients.

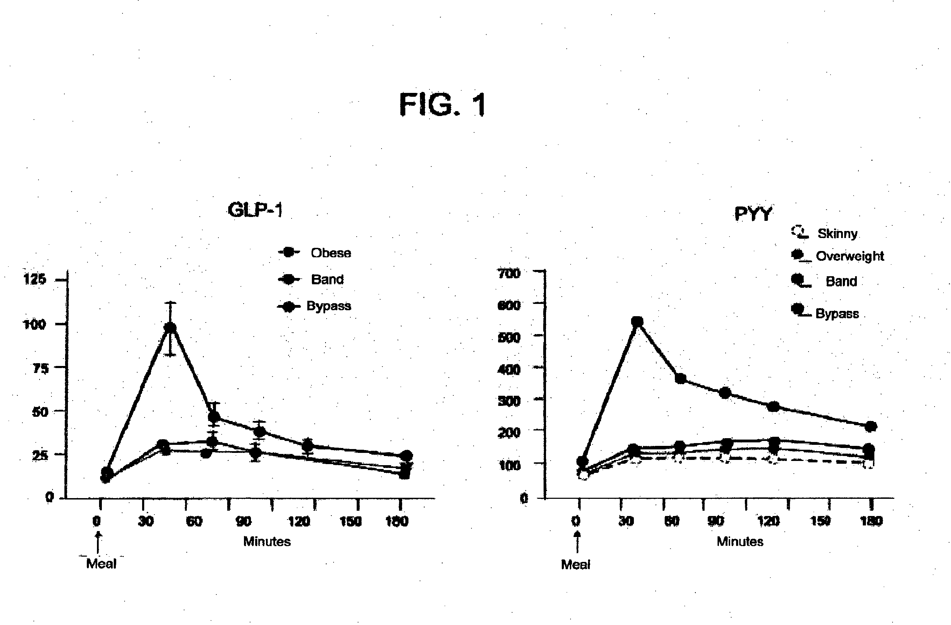

In Figure 1, two plots are presented that show the secretion of the hormones PYY and GLP-1 after the postprandial stimulus in non-operated obese patients, skinny control individuals , those who underwent gastric band surgery and others who underwent roux-en-y gastric bypass (Fobi-Capella or Fobi Pouch Surgery). As noted, individuals undergoing gastric bypass, has a much higher peak secretion of the hormones, about 30 min. After meal, in comparison to the other groups.

The early arrival of these food components, in a state not yet digested or only partially digested, in the terminal ileum (due to the intestinal bypass), stimulates cells in the intestinal mucosa, called "L-CELLS" to secrete supra-physiological amounts of the hormones PYY and GLP-1. Such hormones have physiological actions, defined by several scientific studies such as the appetite inhibition, stimulated energy spending and reduced gastric emptying rate. These actions, shared by the two hormones, have a clear anti-obesity effect. GLP-1 also has a specific additional action called incretinic, which is responsible for stimulating the pancreatic beta cells to produce and secrete insulin. This hormone also inhibits the secretion of Glucagon (hyperglycemic hormone) and may also cause, by A trophic effect, the proliferation of beta cells (hyperplasia). All these actions are beneficial to control Type 2 Diabetes Mellitus.

Albeit these beneficial effects, these invasive surgical procedures markedly alter gastrointestinal anatomy and physiology, bringing about potential risks of mortality, early and late complications, besides several others possible adverse reactions, and finally, high financial costs, both to public and private systems of health insurance, making virtually impossible to reach all the potential patients, because of the massive numbers of obese and type 2 diabetic individuals worldwide.

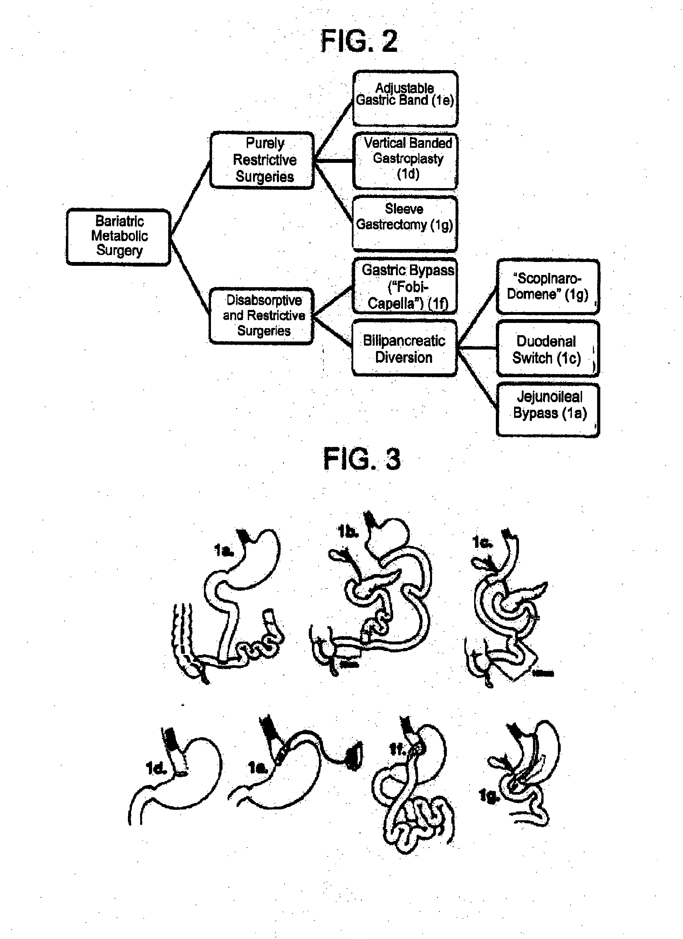

As illustrated in the flowchart of Figure 2 and in images in Figure 3, two groups (sets) of primary techniques are currently practiced, namely: 1 - purely restrictive techniques that ultimately reduce the dimensions of the stomach, creating functional neo-stomachs ranging from 20 to 120 ml of capacity, in contrast to the normal capacity of the intact stomach of 1500 ml, in average. This is intended to restrict, quantitatively, the ingestion of food, as less amounts of food are now necessary to fill UP these new micro-stomachs, up to the physiologic distension limit of its muscular walls. This stimulates tension and distortion receptors in gastric wall, generating electrical signals which are transmitted by nerves (vagus nerves) to the satiety center in the hypothalamus, and produce the sensation of early SATIATION (stop eating) and prolonged satiety (loss of interest in food for several hours); 2 - disabsorptive / restrictive techniques that add gastrointestinal deviations, thereby promoting the arrival of food, not yet digested or only partially digested, in distal parts of the small intestine. This fact, not natural, stimulates supra-physiological or magnified secretion of gastrointestinal hormones, particularly PYY (YY polypeptide) and GLP-1 (glucagon-like polypeptide) which, by their anorexic and incretinic actions, contribute to the success of these surgeries, both in weight loss as in controlling Type 2 Diabetes Mellitus.

In the first group - purely restrictive surgeries -, as shown in the flowchart in Figure 2, there are the following techniques: Adjustable Gastric Band (AGB) (1e), Vertical Banded Gastroplasty (VBG) (1d); and, the most recent one, Sleeve Gastrectomy (1g). The AGB and the VBG surgeries, cited in this group, do not have the benefit of stimulated secretion of these hormones, since the transit of food is natural and the digestive process do not suffer any interference. For Sleeve Gastrectomy, there is some evidence that, by an accelerated gastric emptying, it may also stimulate the release of these hormones, but less than in RYGB. For this reason, these surgeries are the least efficient both for weight loss and for controlling diabetes.

In the second group, of disabsorptive/restrictive surgeries, the techniques practiced are divided into two subgroups, as follows: Gastric Bypass or "Fobi-Capella" (1f), which is the most performed surgery in Brazil and in the world; and the Bile-Pancreatic Diversion as "Scopinaro's" (1b) and Duodenal Switch (1c), and Jejuno-ileal bypass (1a). The latter no longer performed.

In this second group, in general, the food that leaves the stomach, not yet digested, does not go through the proximal small intestine (duodenum and jejunum) and is surgically diverted to the distal portion of the small intestine (distal jejunum and ileum), a fact which stimulates the secretion of hormones mentioned above and even more intensively in Bile-Pancreatic diversion surgeries, because the undigested or partially digested food reaches directly the ileum, the most distal portion of the small intestine, where the concentration of the L-cells producing the said hormones is the highest, releasing them in even higher quantities.

As an al-ternative to the above mentioned surgical practices, the pharmaceutical industry already produced molecules analogous to hormones PYY and GLP-1 and also drugs that inhibit the enzyme DPP-4, responsible for the rapid breakup of these hormones, all aiming to bring about or raise, pharmacologically, their potentially benefic effects. These molecules transformed into drugs have either the disadvantage of high cost or the need for parenteral continuous use or the induction of pharmacological resistance or adverse reactions.

### AIM OF THE INVENTION

The pharmaceutical product in Focus, subject of this application, in tablet forms or pills or mini-pills or micro-granules or in micronized particles, coated by polymers or coating products or any coating substances inclusive the ones appropriated for micro-encapsulation, that are dissolved or disintegrated or broken up in a Ph-dependent fashion, was developed, specifically to release its contents, OF an associative composition of undigested and/or partially digested macronutrients of any kind with or without micro-nutrients and bile salts, delivered to the distal small intestine, especially the ileum and eventually the colon, thereby promoting an amplified stimulus, by means of this new and non-invasive method, for an endogenous supra-physiologic or enhanced secretion of the gut hormones PYY and GLP-1 and may be others, like Oxyntomodulin. By means of the product concerned, anti-obesity, anti-type 2 diabetes mellitus and other beneficial metabolic effects may be obtained, brought about by the anorexigenic and increased energy expenditure actions shared by the two hormones, PYY and GLP-1, and the incretinic action of GLP-1, similarly to what it is obtained by the bariatric-metabolic surgeries described above, but avoiding its adverse reactions and potential early and late complications, arising from the anatomical and physiological changes surgically produced.

After the explanations above, the product will be described in more details in the accompanying drawings:

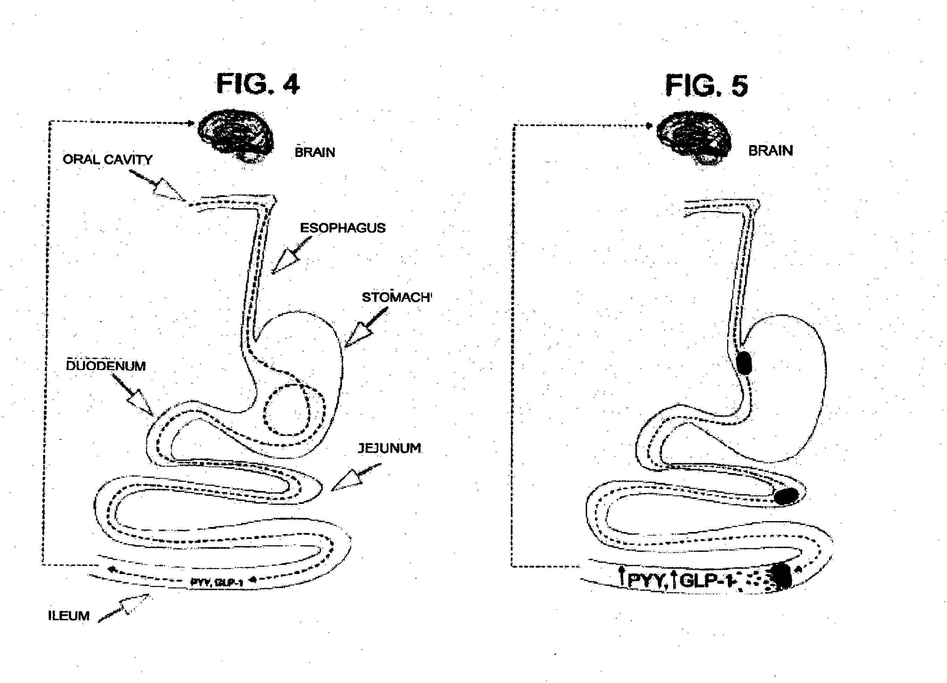

Figure 4 - schematically shows all natural and physiological digestive transit, without the ingestion of this pharmaceutical product, of an individual not subjected to any type of bariatric-metabolic surgery. As noted, upon oral ingestion of food, some salivary enzymes may already start the digestion of some food components, but without significance. The oral pH is approximately 7.0. In the esophagus the normal pH is also 7.0, and in this organ no digestion occurs. In the stomach, where the pH is too acidic (0.9 to 2.0), the gastric juice acts and is responsible for starting the partial breaking up of various protein macromolecules. In the duodenum, initial portion of the small intestine, the content coming from the stomach has a very acidic pH and enters in contact with bile, of pH between 8.0 to 8.5, and the pancreatic juice, of pH between 8.5 and 9.0. This mixture of digestive juices modifies the intra luminal pH in the duodenum to a pH between 5.0 and 5.5. Throughout the jejunum, the pH becomes gradually less acidic and in the ileum (distal portion of the small intestine) the pH ranges from 6.0 to 6.5. When it reaches the distal jejunum and proximal ileum, virtually all macro-nutrients have already been digested and absorbed. Therefore, it is not physiological for the macromolecules OF macronutrients (proteins, lipids, carbohydrates) to reach the ileum, not yet digested or only partially digested. This abnormal fact, if occurs, will stimulate the secretion of large amounts of the hormones PYY and GLP-1, ranging up 4 to 10 times greater than normal, which, through the bloodstream, will reach the arched nucleus in the hypothalamus to produce intense inhibition of appetite and stimulated energy spending. In addition the GLP-1, after reaching the pancreas, stimulates Beta cells to produce and secrete more insulin and inhibits Alfa cells in producing Glucagon.

Figure 5 - schematically shows the path of the pharmaceutical product reason for this patent application.

According to the drawings, the "pharmaceutical product for delivering undigested and/or partially digested macro-nutrients (lipids, proteins and carbohydrates) and/or medium and short chain triglycerides and/or cholesterol and/or bile salts and/or micronutrients to the ileum and/or colon, by means of coating, the whatever effective mixture of these components, by polymers or coating substances or coating derivatives or coating products, that will breakup or dissolve or disintegrate in a pH dependent fashion, aimed to do so, in a pH range between 6,0 to 7,0, to release its contents directly into the ileum and/or colon, stimulating, in this way, the L-cells to release into the bloodstream, supra-physiological or enhanced amounts of gut hormones, mainly and specially GLP-1 and PYY", subject of this invention patent application, consists of an effective associative formula of macro-nutrients like proteins, peptones, polypeptides, lipids, medium and short-chain triglycerides, saturated or insaturated fatty acids, poly and oligosaccharides, bile salts, besides to micronutrients such as vitamins and mineral salts. This formula is prepared in tablet forms or pills or mini-pills or micro-granules or micronized particles, receiving coating or micro-encapsulation with appropriated polymers, such as "Eudragit", for instance, or any coating substances or coating products that will disintegrate or dissolve or broken up in a pH-dependent manner, obtaining the pharmaceutical product in question.

As shown in Figure 5, when ingested, the product so described travels through the esophagus into the stomach where, despite the extremely acidic pH, the polymeric coating, according to the project in question, does not suffer disintegration, travelling through duodenum, jejunum and reaching the ileum. According to the project, after reaching the distal portion of the small intestine, the ileum and/or colon the polymeric coating or coating product or substance will disintegrate or dissolve or breakup by the action of the target pH found in this places, between 6.0 and 7,0, releasing, in the lumen of the ileum and/or colon, the nutrients above described, undigested or partially digested, associated or not with bile salts, in any possible effective combination, contained in this pharmaceutical product. Thus, secretion of the hormones PYY and GLP-1 by L cells is stimulated beyond the physiological range for such cells being present in their greatest concentration in the distal portion of the small intestine (Ileum) and proximal colon. Such effect, once accomplished, results in loss of appetite and satiety feelings by the patient, even before the individual get fed. In addition to this, due to the incretinic action of GLP-1, a better glycemic control in diabetes is achieved.

## Claims

1. pharmaceutical product for delivering of undigested or partially digested macro-nutrients with or without bile salts and/or micronutrients, compressed into tablet forms or pills or min-pills or microgranules or micronized particles, by means of coating or microencapsulating, whatever effective mixture of these components, in any of the above forms, with coating polymers or coating products or substances, that can dissolve or disintegrate or breakup in the intestinal milieu within a pH range from 6,0 to 7,0, releasing its contents into the ileum and/or colon, for enhanced stimulus to the L-cells to secrete PYY and GLP-1 in potentially therapeutic levels", consists of a whatever effective associative formula of macro-nutrients, like proteins, peptones, polypeptides, lipids, medium and short-chain triglycerides, saturated or insaturated fatty acids, cholesterol, poly and oligosaccharides, bile salts, in addition to micronutrients like vitamins and mineral salts, said formulation prepared as above mentioned, after ingestion travels intact through the esophagus into the stomach, duodenum, jejunum and reaches the ileum, **wherein** said pharmaceutical product, only after reaching this distal portion of the small intestine (ileum) have its coating disintegrated or broken up or dissolved by the action of the intestinal milieu with a local pH in the range of 6.0 to 7,0, thus releasing, into the lumen of the ileum and/or colon, the effective mixture of macro and micronutrients and substances as described above, contained in this pharmaceutical product, to stimulate a supra-physiological secretion of the anorexigenic and enhancers of energy expenditure hormones, PYY and, GLP-1, and the incretinic hormone, GLP-1, by the L cells, resulting in inhibition of appetite and satiety feelings by the patient, even before getting fed, in addition to, when stimulating the secretion of insulin (incretinic effect) and inhibiting the secretion of glucagon, allows to a better glycemic control for type 2 diabetic patients.
